# EUROPEAN PATENT SPECIFICATION

(11) **EP 0 539 628 B1**
(45) Date of publication and mention of the grant of the patent: **03.01.1996**
(21) Application number: 91202814.9
(22) Date of filing: 30.10.1991
(51) Int. Cl.: C07C 67/38, C07C 69/54

(54) **Process for the preparation of methacrylate esters**
Verfahren zur Herstellung von Acrylestern
Procédé de préparation d'esters acryliques

(43) Date of publication of application: 05.05.1993
(73) Proprietor: SHELL INTERNATIONALE RESEARCH MAATSCHAPPIJ B.V., NL-2596 HR Den Haag (NL)
(72) Inventor: Hengeveld, Jan, NL-2596 HR The Hague (NL); de Blank, Peter Bastiaan, NL-2596 HR The Hague (NL)

(56) References cited:
- EP-A- 0 392 601

## Description

This invention relates to a process for the preparation of methacrylate esters, wherein a propyne stream is isolated from a mixture of C₃-hydrocarbons essentially consisting of propane, propene, propyne, propadiene and minor amounts of lower and/or higher carbon impurities, and said propyne stream is fed to a carbonylation unit and contacted therein with carbon monoxide and an alcohol in the presence of a carbonylation catalyst to form an alkyl methacrylate.

Such a process, as described in EP-A-392 601, makes an economic use of readily available propyne-containing side-streams from large scale industrial operations in the energy or petrochemical sector, and advantageously avoids the generation of large quantities of waste materials, such as sulphuric acid, ammonium bisulphate and toxic HCN.

These propyne-containing side streams will further contain propadiene, propane and propene as major constituents, of which propadiene can be used in the preparation of the methacrylate, as it is readily isomerised to propyne. A convenient method for selective extraction of propyne from such economic side streams is also described in EP-A-392 601, and includes isomerization of the separated propadiene into propyne for recycling and enhanced utilisation of the C₃H₄-components.

In practice, however, problems were encountered as rapid deactivation of the carbonylation catalyst was observed, even when the propyne feed was carefully freed from any propadiene known to be a poison for the carbonylation catalyst. A further problem was raised by the occurrence of severe fouling of the isomerization and extraction units.

Minor components contained in the propyne-containing side streams used appeared to include hydrocarbons having four or more carbon atoms, such as butadiene, butenes, butynes, pentadienes and pentynes. As these higher carbon components are present rather at the impurity level, it would be expected that they can readily be removed in the subsequent selective propyne extraction or during the final work up of the methacrylate ester product, either as such or as their conversion product, for example 1-butyne having been converted into a 2-ethylpropenoate.

It was found that the presence of the minor amounts of higher carbon impurities in a mixture of C₃-hydrocarbons used in the above-described preparation of methacrylate esters, presents problems much more serious than could be expected on the basis of their molar proportion in the feed alone.

For overcoming the above-indicated problems, it is now proposed that the mixture of C₃-hydrocarbons is subjected to purification for essentially complete removal of hydrocarbon components having four or more carbon atoms.

Conveniently, this purification is effected by distillation. Mixtures of C₃-hydrocarbons of typical composition available for the present process and comprising up to 60 %v of propyne and propadiene combined can be distilled at temperatures in the range of from -10 to 100 °C, preferably of from 20 to 70 °C, and pressures in the range of from 2 to 20 barabs, preferably from 3 to 12 barabs.

In handling streams of hazardous products as propyne and propadiene, it is required that temperatures and pressures are contained within safe limits. Accordingly, it is an advantage of the invention that the distillation is effected on the C₃-hydrocarbon mixture containing the C₃H₄ components diluted with propane and propene. It is preferred that the distillation is effected at molar concentrations of propyne and propadiene together below 50% in the entire space of the distillation apparatus.

In a continuous mode of the present process the propadiene component upon separation is isomerized to its equilibrium mixture with propyne, which is recycled and conveniently combined with the incoming C₃-hydrocarbons feed for extracting additional propyne therefrom. It is also possible to add extraneous and incidental sources of propyne and/or propadiene to the C₃-hydrocarbons feed used in the process. In such cases the content of C₃H₄ components in the C₃-hydrocarbons mixture for distillation may become higher, so that concentrations in the distillation apparatus may exceed 50 %mol.

According to a preferred embodiment of the invention this problem is overcome by diluting the feed to the distillation apparatus with an inert diluent having a volatility equal to or higher than the volatility of propyne. Very suitably, this inert diluent is constituted by propane and/or propene, most suitably their mixture as obtained after extraction of the C₃H₄ components from the original C₃-hydrocarbons feed. This is readily effected by recycling a part of this stream leaving the extraction unit to the distillation apparatus. On a volume basis, from 20 to 70%, preferably from 30 to 60%, most preferably from 40 to 55%, of the propane/propene stream leaving the extraction unit can be recycled to the distillation apparatus.

The C₃-hydrocarbons feed for the present process may be derived from any fossil carbon processing operation, such as refinery operations. Refinery operations particularly envisaged for supplying propyne-containing C₃-mixtures, include ethene crackers, i.e. a type of cracker in which ethene is prepared from hydrocarbon fractions such as naphtha, gas oil, LPG (preferably isobutane) or ethane by thermal cracking; catalytic crackers, i.e. a type of cracker in which hydrocarbons produced by the catalytic cracking of hydrocarbon fractions such as heavy gas oil or vacuum distillates; and LPG-dehydrogenation processes, i.e. a type of process in which propane is converted into propene, either thermally or catalytically. Such processes provide, amongst others, a C₃-stream consisting mainly of C₃-hydrocarbons, in particular propane, propene, propyne and propadiene. The refinery operation may be based on any carbon source, such as coal, crude or natural gas.

Methods for isolating propyne from C₃-mixtures are known, and, for example, described in EP-A-392 601. For example, the concentration of propyne and propadiene may be increased by selective scrubbing with a solvent, suitably being carried out in a column under elevated pressure (2-20, preferably 6-12 bar) using countercurrent flows of an organic solvent and the C₃-mixture (e.g. the bottom effluent of a propane/propene splitter), so that typically a stream consisting essentially of propane and propene (and < 0.2% of propyne/propadiene) is removed as the overhead fraction. From this overhead fraction preferably a stream is split for recycle to and dilution of the distillation of the C₃-hydrocarbons feed, and the remainder is forwarded for further use or processing, usually to the refinery from which the feed of the present process was drawn off. The solvent which absorbs propyne and, at the elevated pressure employed, also propadiene, suitably comprises a polar organic solvent, such as an amide, e.g. dimethylformamide, dimethylacetamide or N-methylpyrrolidone, a nitrile, a sulphone, such as sulfolane, or a mixture thereof.

As propadiene tends to poison catalysts in the carbonylation of propyne and an alcohol, it is selectively removed, for instance by chemical means, such as by isomerization, and/or by physical means such as by distillation, preferably extractive distillation. Preferably, the propadiene is removed by physical means, and subsequently subjected to isomerization for partial conversion into propyne and recycled. It will be appreciated that in this way all of the propyne and propadiene in the original C₃-mixture can in principle be isolated as propyne and used in the carbonylation reaction.

Extractive distillation is a well known method, in which the mixture of propyne and propadiene is dissolved in a polar organic solvent, or available as such a solution, and propadiene is removed as a gas (e.g. by stripping) leaving propyne dissolved in the solvent. Suitable solvents are the same as mentioned hereinabove. The isomerization of propadiene into propyne is well known in the art, and may conveniently be effected in the liquid or gas phase at a temperature in the range of from -30 to 100 °C, preferably 0 to 40 °C, and a pressure in the range of from 0.1 to 100 bar, preferably 1 to 20 bar, in the presence of an isomerization catalyst, for example a catalyst comprising an alkali metal or alkali metal oxide deposited on alumina, or another isomerization catalyst as described in Kirk-Othmer's Encyclopaedia of Chemical Technology, 2nd ed., Volume Supplement (1971), pages 547 to 556, and in US-A-3671605.

After selective removal of propadiene, substantially pure propyne can be isolated from the remaining solution, for example by stripping or flashing. The molar concentration in the propyne stream obtained lies above 80%, preferably above 90%, and most preferably above 99%. It is desirable that the molar ratio of propyne to propadiene in the propyne stream is above 100, preferably above 1000, and most preferably above 5000.

The carbonylation catalyst used in the process according to the invention may be any catalyst having activity for the carbonylation of propyne. It is preferably a Group VIII metal catalyst, more preferably a palladium catalyst. A preferred group of catalysts is disclosed in EP-A-186228, EP-A-271144 and EP-A-386833, and includes catalysts comprising a palladium compound, a ligand (e.g. a monodentate or bidentate phosphine) and an anion of a weakly or non-coordinating acid. Specific examples of such catalysts include combinations of (a) palladium acetate, (b) triphenylphosphine, diphenyl-2-pyridylphosphine, or diphenyl-(6-methyl-2-pyridyl)phosphine, and (c) p-toluenesulphonic acid or trifluoroacetic acid.

The alcohol reacted in the carbonylation step of the present process can be any hydroxy-substituted hydrocarbon, which may or may not carry further substituents. The alcohol is preferably an alkanol having up to 20, more preferably 1 to 4 carbon atoms. Examples of alcohols are methanol, ethanol, propanol, isopropanol, n-butanol, isobutanol, and tert-butanol. Most preferably the alkanol is methanol, thus giving methyl methacrylate as the product.

The carbon monoxide reacted in the carbonylation step of the present process, can be derived from any source, and is preferably used in substantially pure form. Minor amounts of inerts or hydrogen may be present, but increase the need for bleed streams and purification of the end product, and therefore decrease the total yield of the present process, if present at too high concentration.

The reaction between propyne, the alcohol and carbon monoxide is preferably effected at a temperature in the range of from 20 to 200 °C, more preferably 20 to 80 °C, and at a pressure in the range of from 5 to 70 bar. Though a separate solvent may be used, for ease of purification it is preferably absent, since the alcohol precursor and methacrylate ester product constitute suitable liquid vehicles for the carbonylation reaction.

The reactants are preferably fed to the process in substantial accordance with the reaction stoichiometry, i.e. in substantially equimolar amounts apart from any recycle streams. The catalyst system is suitably applied at a concentration corresponding to its activity, for example, to produce 25 kg or more of methyl methacrylate per gram of palladium per hour.

The design and operation of the carbonylation unit and further work up equipment is within the skills of a chemical technologist, and does not require further explanation.

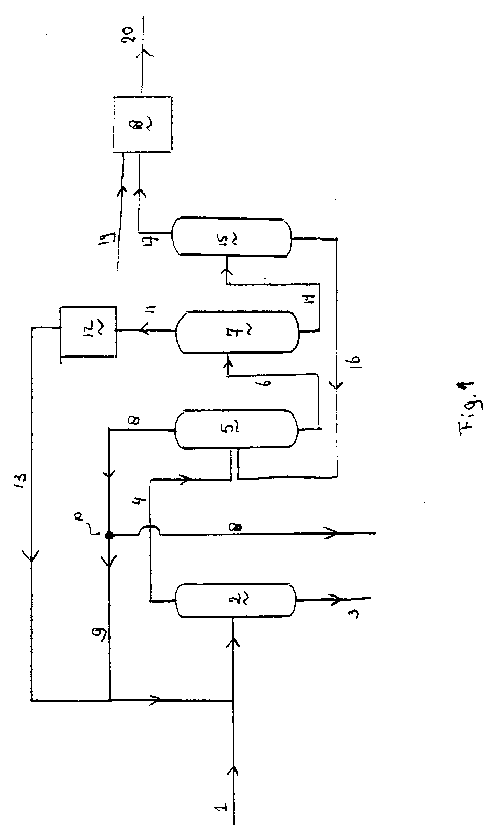

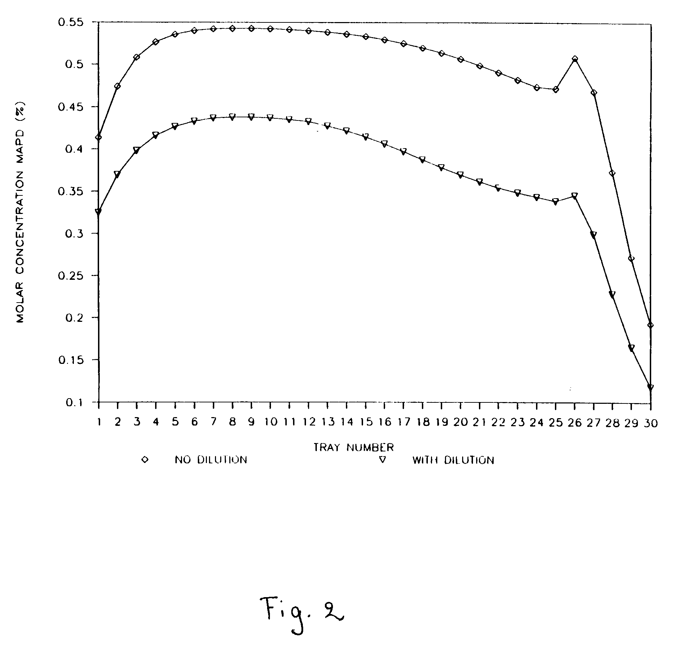

The invention will be further illustrated by the drawings. Herein, Fig. 1 shows a flow scheme of a preferred embodiment of the process of the invention, wherein the C₃-hydrocarbons feed is distilled in the presence of a diluent stream of propane and propene drawn from a propyne/propadiene absorption column, and Fig. 2 shows graphically the effect of dilution on the combined propyne/propadiene molar concentration at the individual trays of a distillation column.

In Fig. 1, a typical C₃-hydrocarbons mixture drawn from an ethene cracker and comprising 29.6 %mol of propyne, 19.9 %mol of propadiene, 32.2 %mol of propane and 14.8 %mol of propene, besides minor amounts of impurities such as butadiene, butenes, butynes, pentenes, pentynes and pentadienes is fed through line 1 to a distillation column operated for example at a pressure of 9 barabs and a temperature of 23 - 67 °C. The bottoms of this column comprises the hydrocarbons having four or more carbon atoms and is removed through 3 for further processing or disposal. Through line 4 an overhead stream comprising the purified feed is forwarded to absorption unit 5, wherein propyne and propadiene are selectively absorbed into a suitable solvent such as dimethylformamide, and the loaded solution is forwarded through line 6 to a first desorption column 7. The gaseous remainder of the feed depleted of propyne and propadiene and mainly consisting of propane and propene, is condensed and drawn off through line 8 for further processing. From line 8, a recycle stream 9 is split at junction 10, and sent to line 1 for combination with the fresh feed. Propadiene desorbed in the first desorption column 7 is sent through line 11 to an isomerization unit 12, wherein it is isomerized to its equilibrium mixture with propyne, which is recycled through line 13 to the fresh feed in line 1. The propyne loaded absorption liquid is sent through 14 to a second desorption column 15, wherein propyne is desorbed. The desorbed solvent is recycled to the absorption column 5 through line 16. The propyne obtained overhead column 15 is sent through line 17 to a carbonylation unit 18, in which further reactants such as carbon monoxide, alcohol, and catalyst are introduced through line 19. The methacrylate ester product is collected through line 20.

Fig. 2 demonstrates the effect of dilution on the C₃-hydrocarbons feed with added isomerized propadiene, in a distillation column of 30 trays having tray No. 1 at its top and tray No. 30 at its bottom. The feed is introduced at tray No. 25. After reaching steady state conditions and without dilution, the concentration of combined propyne and propadiene (MAPD) in the gas phase on most of the trays is above 50 %mol, whereas dilution with a 45 %v bleed stream from the propane/propene overheads of the absorption column decreases the maximum MAPD concentration to below 45 %mol.

## Claims

1. A process for the preparation of methacrylate esters, wherein a propyne stream is isolated from a mixture of C₃-hydrocarbons essentially consisting of propane, propene, propyne, propadiene and minor amounts of lower or higher carbon impurities, and said propyne stream is fed to a carbonylation unit and contacted therein with carbon monoxide and an alcohol in the presence of a carbonylation catalyst to form an alkyl methacrylate, characterized in that the mixture of C₃-hydrocarbons is subjected to purification for essentially complete removal of hydrocarbon components having four or more carbon atoms.

2. The process as claimed in claim 1, wherein said mixture of C₃-hydrocarbons is drawn off from a fossil carbon processing operation.

3. The process as claimed in claim 2, wherein said mixture of C₃-hydrocarbons is drawn off from an ethene cracker, a catalytic cracker or an LPG-dehydrogenation process.

4. The process as claimed in any one or more of claims 1-3, wherein butadiene, butenes and/or butynes are removed from the C₃-hydrocarbon mixture.

5. The process as claimed in any one or more of claims 1-4, wherein the hydrocarbon components having four or more carbon atoms are removed by distillation of the mixture of C₃-hydrocarbons.

6. The process as claimed in claim 5, wherein the distillation is effected at temperatures of from -10 to 100 °C and pressures of from 2 to 20 barabs.

7. The process as claimed in claim 5 or 6, wherein the distillation of the mixture of C₃-hydrocarbons is effected at molar concentrations of propyne and propadiene together below 50% in the entire space of the distillation apparatus.

8. The process as claimed in claim 7, wherein the feed to the distillation apparatus is diluted with an inert diluent having a volatility equal or higher than the volatility of propyne.

9. The process as claimed in claim 8, wherein the diluent is a mixture of propane and propene.

10. The process as claimed in any or more of claims 1-9, wherein the distilled mixture of C₃-hydrocarbons is subjected to extraction of propyne and propadiene, the extract is stripped of propadiene, and propyne is isolated from the propadiene-depleted extract.

11. The process as claimed in any one or more of claims 1-10, wherein the alcohol is an alkanol having 1-4 carbon atoms.

## Patentansprüche

1. Verfahren zur Herstellung von Methacrylsäureestern, wobei ein Propinstrom aus einem C₃-Kohlenwasserstoffgemisch, das im wesentlichen aus Propan, Propen, Propin, Propadien und geringen Mengen an Verunreinigungen mit niedrigerem oder höherem Kohlenstoffgehalt besteht, isoliert wird und dieser Propinstrom einer Carbonylierungsanlage zugeführt und dort mit Kohlenmonoxid und einem Alkohol in Gegenwart eines Carbonylierungskatalysators kontaktiert wird, wobei ein Alkylmethacrylat entsteht, dadurch gekennzeichnet, daß das C₃-Kohlenwasserstoffgemisch zur im wesentlichen vollständigen Entfernung der Kohlenwasserstoffkomponenten mit vier oder mehr Kohlenstoffatomen einer Reinigung unterzogen wird.

2. Verfahren nach Anspruch 1, wobei das C₃-Kohlenwasserstoffgemisch aus einem Verfahren zur Verarbeitung von fossilem Kohlenstoff abgezogen wird.

3. Verfahren nach Anspruch 2, wobei das C₃-Kohlenwasserstoffgemisch aus einer Ethen-Krackanlage, einer katalytischen Krackanlage oder einem LPG-Dehydrierungsverfahren abgezogen wird.

4. Verfahren nach einem oder mehreren der Ansprüche 1-3, wobei aus dem C₃-Kohlenwasserstoffgemisch Butadien, Butene und/oder Butine entfernt werden.

5. Verfahren nach einem oder mehreren der Ansprüche 1-4, wobei die Kohlenwasserstoffkomponenten mit vier oder mehr Kohlenwasserstoffatomen aus dem C₃-Kohlenwasserstoffgemisch destillativ entfernt werden.

6. Verfahren nach Anspruch 5, wobei die Destillation bei Temperaturen von -10 bis 100°C und bei Drücken von 2 bis 20 barabs erfolgt.

7. Verfahren nach Anspruch 5 oder 6, wobei die Destillation des C₃-Kohlenwasserstoffgemischs bei molaren Konzentrationen gesamten Raum des Destillationsapparats durchgeführt wird.

8. Verfahren nach Anspruch 7, wobei das Einsatzgemisch für den Destillationsapparat mit einem inerten Verdünnungsmittel verdünnt wird, dessen Flüchtigkeit gleich der oder höher als die Flüchtigkeit des Propins ist.

9. Verfahren nach Anspruch 8, wobei das Verdünnungsmittel ein Gemisch aus Propan und Propen ist.

10. Verfahren nach einem oder mehreren der Ansprüche 1-9, wobei aus dem destillierten C₃-Kohlenwasserstoffgemisch Propin und Propadien extrahiert wird, aus dem Extrakt das Propadien abgezogen wird und aus dem von Propadien befreiten Extrakt das Propin isoliert wird.

11. Verfahren nach einem oder mehreren der Ansprüche 1-10, wobei der Alkohol ein Alkanol mit 1-4 Kohlenstoffatomen ist.

## Revendications

1. Procédé de préparation d'esters méthacryliques, dans lequel un courant de propyne est isolé d'un mélange d'hydrocarbures en C₃, essentiellement constitué de propane, de propène, de propyne, de propadiène et de quantités mineures d'impuretés carbonées inférieures et/ou supérieures, et ledit courant de propyne est chargé dans une unité de carbonylation et y est mis en contact avec du monoxyde de carbone et un alcool, en présence d'un catalyseur de carbonylation, pour former un méthacrylate d'alkyle, caractérisé en ce que le mélange d'hydrocarbures en C₃ est soumis à une purification pour éliminer pratiquement complètement les composants hydrocarbonés possédant 4 atomes de carbone ou davantage.

2. Procédé selon la revendication 1, dans lequel ledit mélange d'hydrocarbures en C₃ est soutiré d'une opération de traitement de carbures fossiles.

3. Procédé selon la revendication 2, dans lequel ledit mélange d'hydrocarbures en C₃ est soutiré d'un craqueur à éthène, d'un craqueur catalytique ou d'un procédé de déshydrogénation de gaz de pétrole liquéfiés.

4. Procédé selon l'une quelconque des revendications 1-3, dans lequel le butadiène, les butènes et/ou les butynes sont éliminés du mélange d'hydrocarbures en C₃.

5. Procédé selon l'une quelconque des revendications 1-4, dans lequel les composants hydrocarbonés comportant 4 atomes de carbone ou davantage sont éliminés par distillation du mélange d'hydrocarbures en C₃.

6. Procédé selon la revendication 5, dans lequel la distillation est effectuée à des températures de -10 à 100°C et sous des pressions absolues de 2 à 20 bars.

7. Procédé selon la revendication 5 ou 6, dans lequel la distillation du mélange d'hydrocarbures en C₃ est effectuée à des concentrations molaires de propyne et de propadiène, ensemble, inférieures à 50% dans la totalité de l'espace de l'appareil de distillation.

8. Procédé selon la revendication 7, dans lequel la charge de l'appareil de distillation est diluée avec un diluant inerte ayant une volatilité égale ou supérieure à la volatilité du propyne.

9. Procédé selon la revendication 8, dans lequel le diluant est un mélange de propane et de propène.

10. Procédé selon l'une quelconque des revendications 1-9, dans lequel le mélange distillé d'hydrocarbures en C₃ est soumis à une extraction du propyne et du propadiène, l'extrait est débarrassé du propadiène et le propyne est isolé de l'extrait épuisé en propadiène.

11. Procédé selon l'une quelconque des revendications 1-10, dans lequel l'alcool est un alcanol comportant 1-4 atomes de carbone.
